# EUROPEAN PATENT APPLICATION

(11) **EP 2 415 741 A1**
(43) Date of publication of application: **08.02.2012**
(21) Application number: 10758904.6
(22) Date of filing: 29.03.2010
(51) Int. Cl.: C07C 29/149, B01J 31/22, C07C 31/20, C07C 33/20, C07C 33/22, C07C 33/46, C07F 15/00

(54) **METHOD FOR PRODUCING ALCOHOL COMPOUND**

(30) Priority: 31.03.2009 JP 2009084832
(71) Applicant: Sumitomo Chemical Company, Limited, Tokyo 104-8260 (JP)
(72) Inventor: HAGIYA, Koji, Ibaraki-shi Osaka 567-0833 (JP); AOYAGI, Yasutaka, Oita-shi Oita 870-0832 (JP)
(74) Representative: Vossius & Partner
(86) International application number: PCT/JP2010/056109
(87) International publication number: WO 2010/114137

(57) **Abstract**

A method for producing an alcohol compound, wherein a carboxylic acid ester compound is reduced with hydrogen in the presence of a ruthenium complex which is obtained by reacting an imidazolium salt (A) having at least one optionally substituted amino group with a ruthenium compound (B) in the presence of a base (C).

## Description

### TECHNICAL FIELD

The present invention relates to a method for producing an alcohol compound and a ruthenium complex useful in the production method.

### BACKGROUND ART

A method for producing an alcohol compound, wherein a carboxylic acid ester compound is reduced in the presence of a ruthenium complex having phosphorus ligands, is known (See, e.g., Japanese Unexamined Patent Application Publication No. 2001-247499, WO 2008/120475, Japanese Unexamined Patent Application Publication No. 2004-300131, Japanese Unexamined Patent Application Publication (Translation of PCT Application) Nos. 2008-537946 and 2008-538352, and Angew. Chem. Int. Ed., 45, 1113 (2006)).

### DISCLOSURE OF THE INVENTION

The present invention relates to a novel method for producing an alcohol compound and a novel ruthenium complex which is used for the production method.

That is, the present application relates to the following inventions.
[1] A method for producing an alcohol compound, wherein a carboxylic acid ester compound is reduced with hydrogen in the presence of a ruthenium complex which is obtained by reacting an imidazolium salt (A) having at least one optionally substituted amino group with a ruthenium compound (B) in the presence of a base (C).
[2] The production method as described in [1], wherein in the imidazolium salt (A), the optionally substituted amino group is bound to a nitrogen atom in the imidazolium ring through a linking group.
[3] The production method as described in [2], wherein the linking group is an optionally substituted alkylene group.
[4] The production method as described in [1], [2] or [3], wherein the imidazolium salt (A) has a group represented by the formula (1) 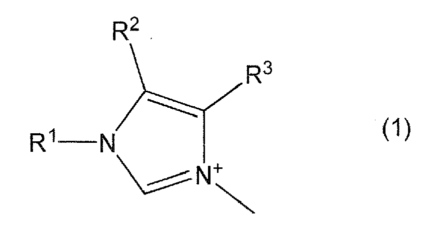 wherein R¹ represents an optionally substituted alkyl group or an optionally substituted aryl group; R² and R³ each independently represent a hydrogen atom, an optionally substituted alkyl group or an optionally substituted aryl group, or R² and R³ are taken together with the carbon atoms to which they are each bound to represent a ring.
[5] The production method as described in [1], [2], [3] or [4], wherein the imidazolium salt (A) has a group represented by the formula (2), as an optionally substituted amino group, 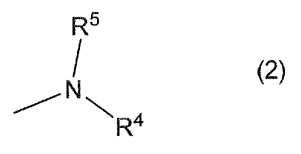 wherein R⁴ and R⁵ each independently represent a hydrogen atom or an alkyl group having 1 to 4 carbon atoms, or R⁴ and R⁵ are taken together to represent an alkylene group having 2 to 8 carbon atoms.
[6] The production method as described in any one of [1] to [5], wherein the imidazolium salt (A) is represented by the formula (3) 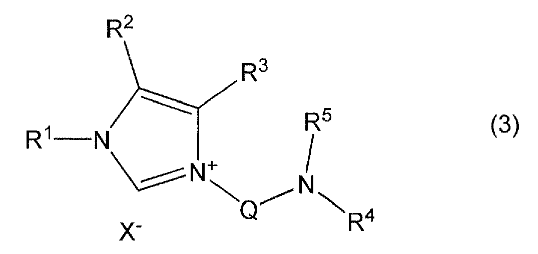 wherein R¹ represents an optionally substituted alkyl group or an optionally substituted aryl group; R² and R³ each independently represent a hydrogen atom, an optionally substituted alkyl group or an optionally substituted aryl group, or R² and R³ are taken together with the carbon atoms to which they are each bound to represent a ring; R⁴ and R⁵ each independently represent a hydrogen atom or an alkyl group having 1 to 4 carbon atoms, or R⁴ and R⁵ are taken together to represent an alkylene group having 2 to 8 carbon atoms. Q represents an optionally substituted alkylene group, and X⁻ represents a monovalent anion.
[7] The production method as described in any one of [1] to [6], wherein the ruthenium compound (B) contains halogens.
[8] The production method as described in any one of [1] to [7], wherein the ruthenium compound (B) is at least one member selected from the group consisting of a compound comprised of halogens and ruthenium, an aromatic compound-coordinated ruthenium dihalide dimer, a diene-coordinated ruthenium dihalide polymer, and a tris(triphenylphosphine)ruthenium compound.
[9] The production method as described in any one of [1] to [8], wherein the base (C) is at least one member selected from the group consisting of an alkali metal alkoxide, an alkali metal hydride and an alkali metal bis(trialkylsilyl)amide.
[10] The production method as described in any one of [1] to [9], wherein the carboxylic acid ester compound is a carboxylic acid ester compound having an aliphatic hydrocarbon group, a carboxylic acid ester compound having an aromatic hydrocarbon group, or a cyclic carboxylic acid ester compound.
[11] The production method as described in any one of [1] to [10], wherein the carboxylic acid ester compound is a compound represented by the formula (6) 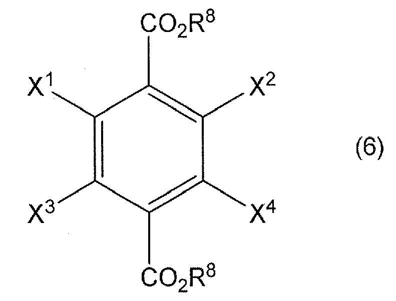 wherein R⁸ represents an alkyl group having 1 to 6 carbon atoms; and X¹, X², X³ and X⁴ each independently represent a hydrogen atom or a halogen atom, with the proviso that at least one of X¹, X², X³ and X⁴ is a halogen atom.
[12] The production method as described in any one of [1] to [11], wherein the amount of the ruthenium complex, which is converted into the amount of ruthenium atoms, is in the range of 0.001 to 0.2 mol per mol of ester of the carboxylic acid ester compound.
[13] The production method as described in [1] to [12], wherein the carboxylic acid ester compound is reduced with hydrogen in the presence of a base.
[14] A ruthenium complex which is obtained by reacting an imidazolium salt (A) having at least one optionally substituted amino group with a ruthenium compound (B) in the presence of a base (C).
[15] A composition containing a ruthenium complex which is obtained by reacting an imidazolium salt (A) having at least one optionally substituted amino group with a ruthenium compound (B) in the presence of a base (C).

### MODE FOR CARRYING OUT THE INVENTION

The present invention will be described hereinafter in detail.

The production method of the present invention is a method wherein a carboxylic acid ester compound is reduced with hydrogen in the presence of a ruthenium complex.

The above ruthenium complex is obtained by reacting an imidazolium salt (A) having at least one optionally substituted amino group and a ruthenium compound (B) in the presence of a base (C).

In the above imidazolium salt (A), the optionally substituted amino group is generally bound to a nitrogen atom or a carbon atom, preferably a nitrogen atom in the imidazolium ring through a linking group.

The above imidazolium ring may be optionally substituted.

Examples of substituents of the above imidazolium ring include optionally substituted alkyl groups and optionally substituted aryl groups.

The above optionally substituted alkyl groups may be linear, branched and cyclic chains, and preferred are alkyl groups having 1 to 20 carbon atoms.

Among the above optionally substituted alkyl groups, unsubstituted alkyl groups include linear or branched alkyl groups having 1 to 20 carbon atoms such as a methyl group, an ethyl group, a propyl group, an isopropyl group, a butyl group, an isobutyl group, a sec-butyl group, a tert-butyl group, a pentyl group and a decyl group; and cycloalkyl groups having 3 to 10 carbon atoms such as a cyclopropyl group, a 2,2-dimethylcyclopropyl group, a cyclopentyl group, a cyclohexyl group and a menthyl group.

Among the above optionally substituted alkyl groups, substituted alkyl groups have optionally substituted aryl groups, optionally substituted alkoxy groups, optionally substituted aryloxy groups, and halogen atoms and the like as substituents. Specifically, the above optionally substituted aryl groups include aryl groups having 6 to 10 carbon atoms such as a phenyl group and a naphthyl group; (C1-C4 alkyl) -substituted aryl groups such as a 2-methylphenyl group and a 4-methylphenyl group; halogen-substituted aryl groups such as a 4-chlorophenyl group; (C1-C4 alkoxy)-substituted aryl groups such as a 4-methoxyphenyl group. The above optionally substituted alkoxy groups specifically include alkoxy groups having 1 to 6 carbon atoms such as a methoxy group, an methoxy group, a propoxy group, an isopropoxy group, a butoxy group, an isobutoxy group, a sec-butoxy group and a tert-butoxy group; haloalkoxy groups having 1 to 6 carbon atoms such as a fluoromethoxy group and a trifluoromethoxy group; a benzyloxy group; (C1-C4 alkyl) -substituted benzyloxy groups such as a 4-methylbenzyloxy group; (C1-C4 alkoxy)-substituted benzyloxy groups such as a 4-methoxybenzyloxy group; phenoxy-substituted benzyloxy groups such as a 3-phenoxybenzyloxy group; (C1-C4 alkoxy)-substituted C1-C4 alkoxy groups such as a methoxymethoxy group, an ethoxymethoxy group and a methoxyethoxy group.

Examples of the above optionally substituted aryloxy groups include a phenoxy group; [(C1-C4 alkyl)-substituted aryl]oxy groups such as a 2-methylphenoxy group and a 4-methylphenoxy group; [(C1-C4 alkoxy)-substituted aryl]oxy groups such as a 4-methoxyphenoxy group; and (phenoxy-substituted aryl)oxy groups such as a 3-phenoxyphenoxy group.

Examples of the above halogen atoms include a fluorine atom and a chlorine atom.

Among the above optionally substituted alkyl groups, substituted alkyl groups include haloalkyl groups such as a fluoromethyl group and a trifluoromethyl group; (C1-C4 alkoxy)-substituted C1-C4 alkyl groups such as a methoxymethyl group, an ethoxymethyl group and a methoxyethyl group; aryl-substituted alkyl groups such as a benzyl group; (halogen-substituted aryl)C1-C4 alkyl groups such as a 4-fluorobenzyl group and a 4-methylbenzyl group; (phenoxy-substituted)C1-C4 alkyl groups such as a phenoxymethyl group.

In each substituent exemplified in this specification, C1-C4 and C3-C10 represent 1 to 4 carbon atoms and 3 to 10 carbon atoms, respectively.

Among the substituents of the above imidazolium ring, optionally substituted aryl groups are preferably optionally substituted aryl groups having 6 to 10 carbon atoms. Among the above optionally substituted aryl groups, unsubstituted aryl groups include aryl groups having 6 to 10 carbon atoms, specifically, a phenyl group and a naphthyl group.

Among the above optionally substituted aryl groups, substituted aryl groups have as substituents, for example, the optionally substituted alkyl groups described above, the optionally substituted alkoxy groups described above and the halogen atoms described above.

Among the above optionally substituted aryl groups, substituted aryl groups include (C1-C4 alkyl)-substituted aryl groups such as a 2-methylphenyl group and a 4-methylphenyl group; halogen-substituted aryl groups such as a 4-chlorophenyl group; and (C1-C4 alkoxy)-substituted aryl groups such as a 4-methoxyphenyl group.

Substituents of the above imidazolium ring are preferably alkyl groups having 1 to 6 carbon atoms, aryl groups having 6 to 10 carbon atoms, a benzyl group, (C1-C4 alkyl)-substituted benzyl groups.

In the above imidazolium salt (A), the linking groups are preferably optionally substituted alkylene groups.

Examples of the alkylene groups in the above linking groups include alkylene groups having 1 to 6 carbon atoms such as a methylene group, an ethylene group, a propylene group, an isopropylene group, a butylene group, an isobutylene group, a pentylene group, an isopentylene group, and a hexylene group.

Substituents which the above alkylene groups can have include optionally substituted aryl groups having 6 to 10 carbon atoms; optionally substituted alkoxy groups having 1 to 6 carbon atoms; optionally substituted aryloxy groups having 6 to 10 carbon atoms; and halogen atoms.

Substituents which the above alkylene groups can have preferably include optionally substituted aryl groups having 6 to 10 carbon atoms such as a phenyl group, a naphthyl group, a 4-methylphenyl group and a 4-methoxyphenyl group; optionally substituted alkoxy groups having 2 to 11 carbon atoms such as a methoxy group, an ethoxy group, a propoxy group, an isopropoxy group, a butoxy group, an isobutoxy group, a sec-butoxy group, a tert-butoxy group, a trifluoromethoxy group, a benzyloxy group, a 4-methylbenzyloxy group, a 4-methoxybenzyloxy group and 3-phenoxybenzyloxy group; optionally substituted aryloxy groups having 6 to 10 carbon atoms such as a phenoxy group, a 2-methylphenoxy group, a 4-methylphenoxy group, a 4-methoxyphenoxy group and a 3-phenoxyphenoxy group; halogen atoms such as a fluorine atom and a chlorine atom; and the like.

The substituted alkylene groups include a fluoromethylene group, a methoxymethylene group, a phenylmethylene group, a fluoroethylene group, a methoxyethylene group, a 2-methoxypropylene group and the like.

In the above imidazolium salt (A), it is preferred that the optionally substituted amino group be bound to one nitrogen atom in the imidazolium ring through a linking group, and it is more preferred that the amino group be bound to one nitrogen atom in the imidazolium ring through a linking group and have a substituent on the other nitrogen in the imidazolium ring.

The above imidazolium salt (A) preferably has, for example, a group represented by the formula (1): 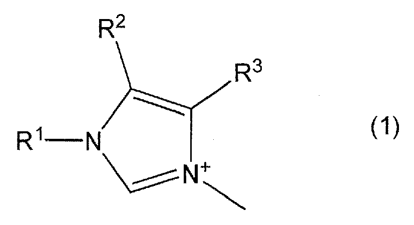 wherein R¹ represents an optionally substituted alkyl group or an optionally substituted aryl group, R² and R³ independently represent a hydrogen atom, an optionally substituted alkyl group or an optionally substituted aryl group, or R² and R³ are taken together with the carbon atoms to which they are each bound to represent a ring.

In the formula (1), the optionally substituted alkyl group and the optionally substituted aryl group each include the groups exemplified as the substituents of the above imidazolium ring.

Examples of the groups represented by taking together with R² and R³ and the carbon atoms include a cyclopentene group, a cyclohexene group, a benzoylene group and a naphthoylene group.

The above optionally substituted amino group is a group in which a hydrogen atom(s) of the amino group (-NH₂) may be substituted with a substituent (s). When two hydrogen atoms are substituted, the amino group may be a cyclic amino group.

Examples of substituents which the above amino group can have include optionally substituted alkyl groups having 1 to 4 carbon atoms. Such alkyl groups include a methyl group, an ethyl group, a propyl group, an isopropyl group, a butyl group, an isobutyl group, a sec-butyl group and a tert-butyl.

The above amino group, for example, includes an amino group represented by the formula (2) (hereinafter may be referred to as amino group (2)): 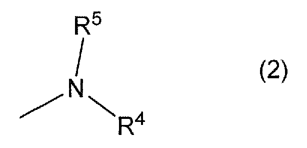 wherein R⁴ and R⁵ each independently represent a hydrogen atom or an alkyl group having 1 to 4 carbon atoms, or R⁴ and R⁵ are taken together to represent an alkylene group having 2 to 8 carbon atoms.

In the above formula (2), the alkyl group having 1 to 4 carbon atoms includes the groups exemplified above.

When R⁴ and R⁵ are taken together to represent an alkylene group having 2 to 8 carbon atoms, the above formula (2) represents a cyclic amino group. Examples of the cyclic amino group include a 1-aziridinyl group, a 1-azetidinyl group, a 1-pyrrolidinyl group and a piperidino group.

Examples of the above imidazolium salt (A) include an imidazolium salt wherein the imidazolium ring has an alkyl group having 1 to 4 carbon atoms on one nitrogen atom and an aminoalkyl group on the other nitrogen atom; an imidazolium salt wherein the imidazolium ring has a (C1-C4 alkyl-substituted)aryl group on one nitrogen atom and an aminoalkyl group on the other nitrogen atom; an imidazolium salt wherein the imidazolium ring has a benzyl group on one nitrogen atom and an aminoal kyl group on the other nitrogen atom; an imidazolium salt wherein the imidazolium ring has an alkyl) group having 1 to 4 carbon atoms on one nitrogen atom and a (C1-C4 alkylamino) alkyl group on the other nitrogen atom; an imidazolium salt wherein the imidazolium ring has a (C1-C4 alkyl-substituted)aryl group on one nitrogen atom and a (C1-C4 alkylamino)alkyl group on the other nitrogen atom; and an imidazolium salt wherein the imidazolium ring has a benzyl group on one nitrogen atom and a (C1-C4 alkylamino)alkyl group on the other nitrogen atom.

Specific examples of the above imidazolium salt (A) include a 1-methyl-3-butyl-4-(1-aminomethyl)imidazolium salt, a 1-methyl-3-butyl-5-(2-aminoethyl)imidazolium salt, a 1,3-dimethyl-4-(3-aminopropyl)imidazolium salt, a 1-benzyl-5-(3-aminopropyl)imidazolium salt and a 5-amino-1,3-dimethyl-1H-benzimidazolium salt.

The above imidazolium salt (A) also includes a salt represented by the formula (3) (hereinafter this compound may be referred to as imidazolium salt (3)): 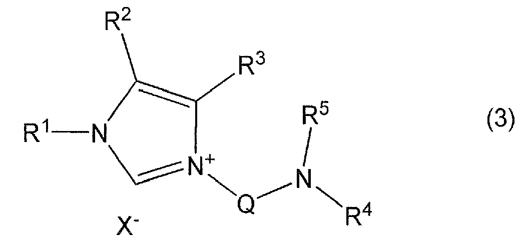 wherein R¹, R², R³, R⁴ and R⁵ each have the same meanings as described above; Q represents an optionally substituted alkylene group, and X⁻ represents a monovalent anion.

In the formula (3), the alkylene group includes the alkylene groups exemplified as the above linking group.

Examples of X⁻ include halide ions such as a chloride ion, a bromide ion and an iodide ion; alkanesulfonate ions such as metilaitesulfouate; alkanesulfonate ions having fluorine atoms such as trifluoromethanesulfonate; an acetate ion; acetate ions having halogen atoms such as trifluoroacetate and trichloroacetate ions; a nitrate ion; a perchlorate ion; tetrahaloborate ions such as tetrafluoroborate and tetrachloroborate; hexahalophosphate ions such as hexafluorophosphate; hexahaloantimonate ions such as hexafluoroantimonate and hexachloroantimonate; pentahalosutannate ions such as pentafluorosutannate and pentachlorostannate; optionally substituted tetraarylborates such as tetraphenylborate, tetrakis(pentafluorophenyl)borate and tetrakis[3,5-bis(trifluoromethyl)phenyl]borate; and the like. Among these, halide ions are preferred.

In the above imidazolium salt (3), it is preferred that R¹ be a benzyl group having as substituents an alkyl group having 1 to 4 carbon atoms, a benzyl group, an aryl group and an alkyl group having 1 to 4 carbon atoms; R² be a hydrogen atom; R³ be a hydrogen atom or an alkyl group having 1 to 4 carbon atoms; and R⁴ and R⁵ be a hydrogen atom or a methyl group.

Specifically, the above imidazolium salt (3) includes 1-methyl-3-(1-aminomethyl)imidazolium bromide, 1-ethyl-3-(1-aminomethyl)imidazolium bromide, 1-butyl-3-(1-aminomethyl)imidazolium bromide, 1-benzyl-3-(1-aminomethyl)imidazolium bromide, 1-[(2,4,6-trimethylphenyl)methyl]-3-(1-aminomethyl)imidazoli um bromide, 1-methyl-3-(2-aminoethyl)imidazolium bromide, 1-butyl-3-(2-aminoethyl)imidazolium bromide, 1-phenyl-3-(2-aminoethyl)imidazolium bromide, 1-benzyl-3-(2-aminoethyl)imidazolium bromide, 1-[(2,4,6-trimethylphenyl)methyl]-3-(2-aminoethyl)imidazoliu m bromide, 1-methyl-3-(3-aminopropyl)imidazolium bromide, 1-ethyl-3-(3-aminopropyl)imidazolium bromide, 1-benzyl-3-(3-aminopropyl)imidazolium bromide, 1--[(2,4,6-trimethylphenyl)methyl]-3-(3-aminopropyl)imidazoli um bromide, 1-methyl-3-(4-aminobutyl)imidazolium bromide, 1-ethyl-3-(4-aminobutyl)imidazolium bromide, 1-butyl-3-(4-aminobutyl)imidazolium bromide, 1-benzyl-3-(4-aminobutyl)imidazolium bromide, 1-[(2,4,6-trimethylphenyl)methyl]-3-(4-aminobutyl)imidazoliu m bromide, 3-[(3-dimethylamino)propyl]-1-methyl-1H-imidazolium bromide, 3-[(3-dimethylamino)propyl]-1-butyl-1H-imidazolium bromide, 3-[(3-dimethylamino)propyl]-1-benzyl-1H-imidazolium bromide, 3-[(3-diethylamino)propyl]-1-benzyl-1H-imidazolium bromide, 3-[(3-methylami_no)propyl]-1-benzyl-1H-imidazolium bromide, 3-[(3-dibutylamino)propyl]-1-benzyl-1H-imidazolium bromide, 3-[(3-dimethylamino)-2-methylpropyl]-1-benzyl-1H-imidazolium bromide, 3-[(4-dimethylamino)butyl]-1-methyl-1H-imidazolium bromide, 3-[(4-methylamino)butyl]-1-butyl-1H-imidazolium bromide, 3-[(4-dimethylamino)butyl]-1-benzyl-1H-imidazolium bromide, 3-[(2-dimethylamino)ethyl]-1-methyl-1H-imidazolium bromide, 3-[(2-dimethylamino)ethyl]-1-butyl-1H-imidazolium bromide, 3-[(2-dimethylamino)ethyl]-1-benzyl-1H-imidazolium bromide, and compounds in which the "bromide"s of the above compounds are replaced with "chloride", "iodide", "methanesulfonate", "trifluoromethanesulfonate", "nitrate", "perchlorate", "tetrafluoroborate", "tetrachloroborate", "hexafluorophosphate", "hexafluoroantimonate", "hexachloroantimonate", "pentafluorostannate", "pentachlorostannate", "tetraphenylborate", "tetrakis(pentafluorophenyl)borate", and "tetrakis[3,5-bis(trifluoromethyl)phenyl]borate", respectively. In imidazolium salt (3), an optionally substituted amino group moiety may be a cation. Hydrogen halides such as hydrogen chloride and hydrogen bromide and mineral acids such as sulfuric acid and phosphoric acid can be counter ions of the cation.

The above imidazolium salt (A) can be, for example, prepared by a conventional method such as alkylation of a compound having an amino group and an imidazole group, and by replacing a leaving group with an amino group in an imidazolium salt having as a substituent an alkylene group bound to the leaving group.

The above compound having an amino group and an imidazole group includes histamine, α-methylhistamine and homohistamine and the like. The above leaving group includes halogen atoms such as chlorine and bromine, alkylsulfonyloxy groups such as a methanesulfonyloxy group and an ethanesulfonyloxy group, and the like. Substitution of the above leaving group with an amino group can be made by a known method.

Preparation of the imidazolium salt (3) will be illustrated as a preparation example of the above imidazolium salt (A).

A method for producing the above imidazolium salt (3) include a method reacting a compound represented by the formula (10) : 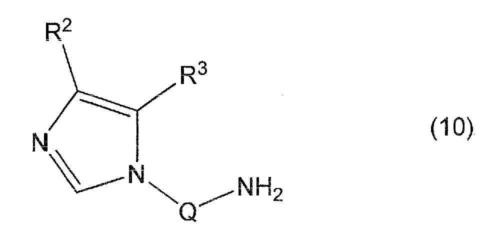 wherein R², R³ and Q represent the same meanings as described above;
with a benzaldehyde or the like to convert -NH₂ of the imidazole compound into a Schiff base, and reacting the obtained base compound with a compound represented by the formula (11):

**R¹-Z** (11)

wherein R¹ represents the same meaning as described above. Z represents a chlorine atom, a bromine atom or an iodine atom; to obtain an imidazole salt, and then converting the Schiff base of the imidazole salt into an amino group to obtain the imidazolium salt (3) (See, e.g., Polyhedron, 23, 2821 (2004)).

The imidazolium salt (3), wherein R⁴ and R⁵ both are hydrogen atoms and X⁻ is Z⁻, can be obtained by such a method. Imidazolium salt (3) with different structures can also be produced from the imidazolium Salt (3) obtained by the above method by substituting a hydrogen atom (s) of -NH₂ with an alkyl group (s) by a conventional method, and by replacing a monovalent anion by a conventional method.

As a preparation method, the above imidazolium salt (3) can be produced by reacting a compound represented by the formula (9) : 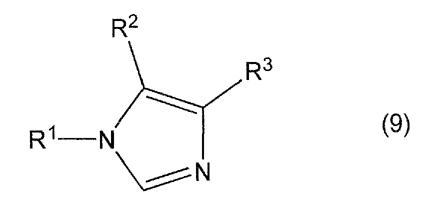 wherein R¹, R² and R³ represent the same meanings as described above;
with a compound represented by the formula (12): 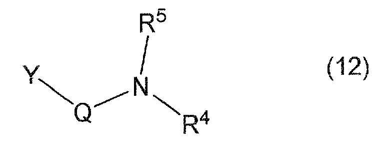 wherein R⁴, R⁵ and Q represent the same meanings as described above, and Y represents a chlorine atom, a bromine atom or an iodine atom (See, e.g., Organometallics, 27, 224 (2008)).

The compounds represented by each of the above formulae (9) to (12) may be those which are commercially available or which are synthesized by any known method. Methods for producing the compounds represented by the above formulae (9) and (10) are described in, e.g., the specification of U.S. Patent No. 4619941.

Next, a ruthenium complex which is obtained by reacting the above imidazolium salt (A) with a ruthenium compound (B) in the presence of a base (C) (hereinafter may be referred to as ruthenium complex), and a composition containing the ruthenium complex will be described.

The above ruthenium complex can be suitably used as a catalyst for reducing an carboxylic acid ester compound described below. The above ruthenium complex can be produced at a low cost since it is obtained from the above imidazolium salt (A), ruthenium compound (B) and base (C).

The ruthenium compound (B) preferably contains halogens. Ruthenium containing halogens includes compounds comprised of halogens and ruthenium such as ruthenium(III) chloride and ruthenium(III) bromide; aromatic compound-coordinated ruthenium dihalide dimers such as (p-cymene)ruthenium dichloride dimer, (benzene)ruthenium dichloride dimer, (mesitylene)ruthenium dichloride dimer, (hexamethylbenzene)ruthenium dichloride dimer, and (p-cymene)ruthenium dibromide dimer; diene-coordinated ruthenium dihalide polymers such as ruthenium 1,5-cyclooctadiene dichloride polymer, and ruthenium dinorbornadiene dichloride polymer; tris(triphenylphosphine)ruthenium halides such as tris(triphenylphosphine)ruthenium dichloride, tris(triphenylphosphine)ruthenium dibromide, tris(triphenylphosphine) ruthenium hydrochloride; and the like. These ruthenium compounds may be hydrates.

As the above ruthenium compound (B), aromatic compound-coordinated ruthenium dihalide dimers, compounds comprised of halogens and ruthenium and tris(triphenylphosphine)ruthenium halides are more preferred.

The above ruthenium compound (B) may be used individually, or two or more may be used. A ruthenium compound (B) may be one which is commercially available or which is synthesized by a known method.

The amount of a ruthenium compound (B), which is the amount of ruthenium atoms in the ruthenium compound (B), is used in the range of generally 0.3 to 2 mol, preferably 0.3 to 1 mol, and further preferably 0.4 to 0. 6 mol per mol of the imidazolium salt (A).

The amount of ruthenium atoms in the ruthenium compound (B) is determined by known means such as element analysis using ICP emission spectroscopy.

A base (C) can generally generate a carbene by extracting a hydrogen atom, which is bound to the carbon atom at 2-position of an imidazolium ring. When the base (C) is brought into contact with, for example, a compound having a group represented by the formula (1), the hydrogen atom at 2-position on the imidazolium ring is extracted and a group represented by the formula (1'): 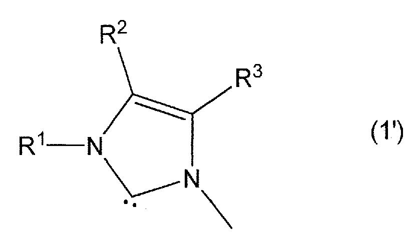 (wherein R¹, R² and R³ each represent the same meanings as described above; and : indicates that such a carbon atom is a carbene) can be generated.

Examples of the above base (C) include alkali metal alkoxides such as sodiummethoxide, sodium ethoxide and potassium tert-butoxide; alkali metal hydrides such as lithium hydride, sodium hydride and potassium hydride; alkali metal bis(trialkylsilyl)amides such as sodium bis(trimethylsilyl)amide and potassium bis(trimethylsilyl)amide; and the like, and alkali metal-containing compounds are preferred and alkali metal hydrides are more preferred. The bases (C) may be used individually, or two or more may be used. A base (C) may be one which is commercially available or which is synthesized by a known method.

The amount to be used of the base (C) is in the range of generally 0.8 to 3 mol, and preferably 1 to 2 mol per mol of the imidazolium salt (A).

When the amino group of the imidazolium salt (A) form a salt with hydrogen halide, mineral acid or the like, the amount to be used of the base (C) may be suitably determined in view of neutralization of the salt. When the amino group is, for example, a monohydrochloride, the amount of the base (C) may be used 1 more mol of the above range. That is, the amount to be used of the base (C) in this case is the range of generally 1.8 to 4 mol, and preferably 2 to 3 mol per mol of the imidazolium salt (A).

A synthesis of a ruthenium complex is generally carried out in the presence of an organic solvent.

Examples of such an organic solvent include ether solvents such as methyl tert-butyl ether, tetrahydrofuran, dimethoxyethane, and diglyme; nitrile solvents such as acetonitrile and propionitrile; aromatic hydrocarbon solvents such as toluene and xylene; amide solvents such as dimethylformamide, dimethylacetoamide and N-methyl-2-pyrrolidone; and the like, and preferred are ether solvents and amide solvents.

The amount to be used of the organic solvent is preferably 1 part by weight or more and 100 parts by weight or less per part by weight of the imidazolium salt (A) in view of productivity.

In the synthesis of a ruthenium complex, the procedure is not restricted, and the synthesis can be, for example, performed by mixing the imidazolium salt (A) and the base (C), and adding the ruthenium compound (B) to the mixture.

The reaction temperature for synthesizing a ruthenium complex is generally in the range of-20° to 100°C, and preferably 0° to 60°C. The progress of such synthesis reaction can be confirmed by general analytical means such as high performance liquid chromatography, thin-layer chromatography, NMR and IR.

In the reaction mixture obtained by reaction of the imidazolium salt (A) with the ruthenium compound (B), a ruthenium complex is contained. The reaction mixture, per se, or the reaction mixture on which isolation and purification are performed can be used for reduction described below.

The isolation includes, for example, washing, separation, crystallization and concentration. The purification includes recrystallization, column chromatography and the like. The above purification is generally carried out after isolation. In the present invention, the ruthenium complex which is isolated from the reaction mixture is preferred. For example, when an insoluble matter such as an alkali metal halide is deposited in the reaction mixture, it is preferred that the insoluble matter be removed from the reaction mixture by filtration and the like.

Since the above ruthenium complex is obtained from the above imidazolium salt (A), ruthenium compound (B) and base (C), it generally has a structure in which a ruthenium metal is coordinated with an imidazole ring and an amino group derived from the imidazolium salt (A), and a group derived from the ruthenium compound (B). When the ruthenium compound (B) contains halogens, a ruthenium complex having halogen atoms can be obtained. Even when a ruthenium compound having phosphorus ligands, like tris(triphenylphosphine) ruthenium halide, is used as the ruthenium compound (B), a ruthenium complex not having triphenylphosphine can be obtained by removing the phosphorus ligands by crystallization operation and the like.

A composition containing the above ruthenium complex includes a reaction mixture obtained by the reaction of the imidazolium salt (A) with the ruthenium compound (B), and a powder or a solution containing a ruthenium complex isolated from the reaction mixture. Since the composition contains the ruthenium complex, it can be suitably used for producing a carboxylic acid ester compound described below.

Next, a method for producing an alcohol compound, wherein a carboxylic acid ester compound is reduced with hydrogen in the presence of a ruthenium complex, will be described.

The above carboxylic acid ester compound is an organic compound having one or more esters. The above carboxylic acid ester compound may be a monoester and a compound having multiple esters such as a diester. The above compound having multiple esters includes an oxalic acid diester, a malonic acid diester, a phthalic acid diester, a maleic acid diester, a glutaric acid diester and an adipic acid diester.

The above carboxylic acid ester compound includes a carboxylic acid ester compound having an aliphatic hydrocarbon group (hereinafter this compound is referred to as "aliphatic hydrocarbon-containing carboxylic acid ester"), a carboxylic acid ester compound having an aromatic hydrocarbon group (hereinafter this compound is referred to as "aromatic hydrocarbon-containing carboxylic acid ester"), and a cyclic carboxylic acid ester compound.

In the above carboxylic acid ester compounds, the above aliphatic hydrocarbon group includes optionally substituted alkyl groups and optionally substituted alkenyl groups.

Examples of such alkyl groups include linear, branched or cyclic alkyl groups having 1 to 20 carbon atoms such as a methyl group, an ethyl group, a propyl group, an i.sopropyl group, a butyl group, an isobutyl group, a sec-butyl group, a tert-butyl group, a pentyl group, a decyl group, a cyclopropyl group, a 2,2-dimethylcyclopropyl group, a cyclopentyl group, a cyclohexyl group and a methyl group. Examples of substituents of the alkyl groups include halogen atoms such as a fluorine atom; alkoxy groups having 1 to 4 carbon atoms such as a methoxy group and an ethoxy group; aryl groups such as a phenyl group and a naphthyl group; aryloxy groups such as a phenoxy group, a 1-naphthyloxy group and a 2-naphthyloxy group; an amino group; a hydroxyl group; carbonyloxyalkyl groups having 2 to 5 carbon atoms. Specific examples of the substituted alkyl groups include substituted alkyl groups having 1 to 20 carbon atoms such as a fluoromethyl group, a trifluoromethyl group, a methoxymethyl group, an ethoxymethyl group, a methoxyethyl group, a hydroxymethyl group, an aminomethyl group and a benzyl group. Examples of such alkenyl groups include linear, branched or cyclic alkenyl groups having 2 to 12 carbon atoms such as an ethenyl group, a 1-propenyl group, a 1-methylethenyl group, a 1-methyl-2-propenyl group, a 1-butenyl group, a 2-butenyl group, a 3-butenyl group, a 1-methyl-1-propenyl group, a 2-methyl-1-propenyl group, a 1-pentenyl group, a 2-pentenyl group, a 1-hexenyl group, a 1-decenyl group, a 1-cyclopentenyl group and a 1-cyclohexenyl group. Examples of substituents of the alkenyl groups include halogen atoms such as a fluorine atom, a chlorine atom and a bromine atom; alkoxy groups such as a methoxy group and an ethoxy group; aryl groups such as a phenyl group, a 1-naphthyl group and a 2-naphthyl group; aryloxy groups such as a phenoxy group; an amino group; a hydroxyl group; and the like. Specific examples of the substituted alkenyl groups include a 3-fluoro-1-propenyl group, a 3-methoxy-1-propenyl group and a 3-phenoxy-1-butenyl group.

Specific examples of the above aliphatic hydrocarbon-containing carboxylic acid ester include ethyl acetate, methyl propionate, isopiopyl butanoate, octyl pentanoate, benzyl hexanoate, pentyl heptanoate, methyl octanoate, benzyl cyclohexanecarboxylate, benzyl pivalate, butyl tert-butyl acetate, ethyl acrylate, ethyl 3,3-dimethyl-2-(2-methyl-1-propenyl)cyclopropanecarboxylate, benzyl 3,3-dimethyl-2-(2-methyl-1-propenyl)cyclopropanecarboxylate, methyl 3,3-dimethyl-2-(1-propenyl)cyclopropanecarboxylate, heptyl pyruvate, dimethyl oxalate, diethyl malonate, dipropyl glutarate, and dibutyl adipate.

The above aliphatic hydrocarbon-containing carboxylic acid ester may be one which is commercially available or which is produced by a known method.

In the above carboxylic acid ester compounds, the above aromatic hydrocarbon group includes optionally substituted aryl groups.

Examples of such aryl groups include aryl groups having 6 to 10 carbon atoms such as a phenyl group, a 1-naphthyl group and a 2-naphthyl group. Examples of substituents of the aryl groups include the optionally substituted alkyl groups described above; the optionally substituted alkenyl groups described above; aryl groups such as a phenyl group, a 1-naphthyl group and a 2-naphthyl group; halogen atoms such as a fluorine atom, a chlorine atom and a bromine atom; alkoxy groups such as a methoxy group and an ethoxy group; an amino group; a hydroxyl group; carbonyloxyalkyl groups; and the like. Specific examples of the substituted aryl groups include a 2-methylphenyl group, a 4-chlorophenyl group, a 4-methylphenyl group, a 4-methoxyphenyl group, a 4-aminophenyl group, a 4-hydroxyphenyl group, a 3-phenoxy-1-butenyl group and a styryl group.

The above aromatic hydrocarbon-containing carboxylic acid ester includes, for example, a compound represented by the formula (6): 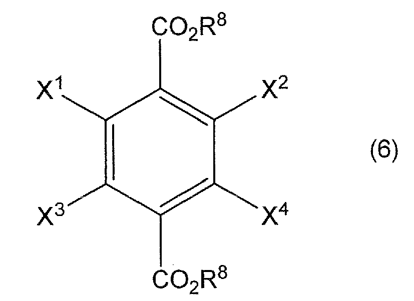 wherein R⁸ represents an alkyl group having 1 to 6 carbon atoms; X¹, X², X³ and X⁴ each independently represent a hydrogen atom or a halogen atom, with the proviso that at least one of X¹, X², X³ and X⁴ is a halogen atom.

The alkyl group having 1 to 6 carbon atoms represented by R⁸ includes a methyl group, an ethyl group, a propyl group, a butyl group, a pentyl group and a hexyl group.

Specific examples of the above aromatic hydrocarbon-containing carboxylic acid ester include ethyl cinnamate, (1-octyl)cinnamate, benzyl phenylacetate, methyl benzoate, isopropyl benzoate, methyl 2-fluorobenzoate, benzyl 2-fluorobenzoate, methyl 2-chlorobenzoate, ethyl 2-bromobenzoate, propel 3-fluorobenzoate, butyl 3-chlorobenzoate, pentyl 3-bromobenzote, methyl 4-fluorobenzoate, methyl 4-aminobenzoate, methyl 4-bromobenzoate, benzyl 2,4-difluorobenzoate, ethyl 2,4-dichlorobenzoate, methyl 3,5-difluorobenzoate, methyl 2,3,5,6-tetrafluorobenzoate, methyl 4-methyl-2,3,5,6-tetrafluorobenzoate, methyl 3-phenoxybenzoate, methyl 4-methyl benzoate, methyl 3-trifluoromethyl benzoate, methyl 2-methoxybenzoate, methyl 4-phenylbutyrate, methyl 3-(4-hydroxyphenyl)propanoate, 1-methoxycarbonylnaphthalene, dimethyl phthalate, diethyl isophthalate, dimethyl terephthalate, dimethyl 3,4,5,6-tetrafluorophthalate, diethyl 2,4,5,6-tetrafluoroisophthalate, dimethyl terephthalate, dimethyl 2-fluoroterephthalate, dimethyl 2-chloroterephthalate, dimethyl 2,5-difluoroterephthalate, dimethyl 2,6-difluoroterephthalate, dimethyl 2,3-difluoroterephthalate, dimethyl 2,5-dichloroterephthalate, dimethyl 2,6-dichloroterephthalate, dimethyl 2,3-dichloroterephthalate, dimethyl 2,3,5-trifluoroterephthalate, dimethyl 2,3,5-trichloroterephthalate, dimethyl 2,3,5,6-tetrafluoroterephthalate, diethyl 2,3,5,6-tetrafluoroterephthalate, dipropyl 2,3,5,6-tetrafluoroterephthalate, diisopropyl 2,3,5,6-tetrafluoroterephthalate, dibutyl 2,3,5,6-tetrafluoroterephthalate, di tert-butyl 2,3,5,6-tetrafluoroterephthalate, dimethyl 2,3,5,6-tetrachloroterephthalate, diethyl 2,3,5,6-tetrachloroterephthalate, dipropyl 2,3,5,6-tetrachloroterephthalate, diisopropyl 2,3,5,6-tetrachloroterephthalate, dibutyl 2,3,5,6-tetrachloroterephthalate, di tert-butyl 2,3,5,6-tetrachloroterephthalate, dipentyl 2,3,5,6-tetrachloroterephthalate, dihexyl 2,3,5,6-tetrachloroterephthalate, and dimethyl 2,3,5-trifluoro-6-chloroterephthalate,

The above aromatic hydrocarbon-containing carboxylic acid ester can be produced according to, for example, a method in which a corresponding acid halide is reacted with an alcohol (See, e. g. , Japanese Examined Patent Application Publication No. Hei-4-66220).

In the above carboxylic acid ester compounds, a cyclic carboxylic acid ester compound includes optionally substituted lactones.

Such lactones are preferably 4- to 22- membered rings. It is preferred that the above lactone be a ring structure containing an optionally substituted alkylene group having 2 to 20 carbon atoms. Such alkylene groups include an ethylene group, a propylene group, an isopropylene group, a butylene group, a pentylene group, a hexylene group, a heptalene group, an octalene group and a decylene group. Examples of substituents of the alkylene groups include halogen atoms such as a fluorine atom; alkoxy groups such as a methoxy group and an ethoxy group; aryl groups such as a phenyl group and a naphthyl group; aryloxy groups such as a phenoxyl group, a 1-naphthyloxy group and a 2-naphthyloxy group; alkenyl groups such as an ethenyl group, a 1-propenyl group, a 1-methylethenyl group, a 1-butenyl group, a 1-methyl-1-propenyl group, a 2-methyl-1-propenyl group, a 1-pentenyl group, a 1-hexenyl group and a 1-decenyl group; an amino group; a hydroxyl group; and the like. Specific examples of substituted alkylene groups include a fluoroethylene group, a methoxymethylene group, a 2-hydroxypropylene group, a 2-aminobutylene group, a 2-phenylmethylbutylene group and the like.

The above cyclic carboxylic acid ester compounds include β-propiolactone, γ-butyrolactone, δ-valerolactone, ε-caprolactone, β-methyl-ε-caprolactone, γ-methyl-ε-caprolactone, heptanolactone, octanolactone, nonanolactone, decanolactone and the like. A cyclic carboxylic acid ester compound may be one which is commercially available or which is produced by a known method.

Hydrogen gas is generally used as hydrogen for reduction of a carboxylic acid ester compound. Commercially available products can be used as the hydrogen gas. In the reduction, hydrogen can be generally supplied in the range of pressure between 0.1 and 5 MPa.

In the reduction, hydrogen is generally used in the range of 1 to 100 mol per mol of the carboxylic acid ester compound.

It is preferred that the amount to be used of the above ruthenium complex, which is converted into the amount of ruthenium atoms, be in the range of preferably 0.001 to 0.2 mol, and more preferably 0.01 to 0.2 mol per mol of ester of the carboxylic acid ester compound.

In the above ruthenium complex, the amount of ruthenium atoms can be measured by a known method such as element analysis using ICP emission spectroscopy.

When the ruthenium complex has halide ions, it is preferred that the carboxylic acid ester compound be reduced in the presence of a base. In this case, since halide ions are removed, the catalytic activity of the ruthenium complex can be improved.

Examples of the base in the above reduction include alkali metal hydroxides such as lithium hydroxide, sodium hydroxide and potassium hydroxide; alkaline-earth metal hydroxides such as magnesium hydroxide and calcium hydroxide; alkali metal alkoxides such as sodium methoxide, sodium ethoxide and potassium tert-butoxide; and alkali metal hydrides such as lithium hydride, sodium hydride and potassium hydride. The bases may be the same kind or a different kind of the base (C) described above, and alkali metal hydroxides and alkaline-earth metal hydroxides are preferred, and alkali metal hydroxides are more preferred.

The amount to be used of the base in the above reduction is in the range of generally 1 to 100 mol, preferably 1 to 10 mol and more preferably 1 to 5 mol per mol of halide ion contained in the ruthenium complex.

The above reduction is preferably carried out in the presence of an organic solvent. Examples of such an organic solvent include ether solvents such as methyl tert-butyl ether, tetrahydrofuran, dimethoxyethane and diglyme; alcohol solvents such as methanol, ethanol and isopropanol; aromatic hydrocarbon solvents such as toluene and xylene; and aliphatic hydrocarbon solvents such as hexane, heptane and cyclohexane; and ether solvents and aromatic hydrocarbon solvents are preferred.

The amount to be used of the organic solvent is 1 part by weight or more and 100 parts by weight or less per part by weight of the carboxylic acid ester compound in view of productivity.

The above reduction is, for example, carried out by mixing a carboxylic acid ester compound, a ruthenium complex and, if needed, an organic solvent and/or a base, and then replacing the inside of a device for the above reduction with hydrogen to adjust hydrogen pressure and reaction temperature.

In the above reduction, the reaction temperature is in the range of generally 0° to 200°C, and preferably 50° to 180°C. In the above reduction, the reaction pressure is in the range of generally 0.1 to 5 MPa, and preferably 0.5 to 5 MPa. The progress of the reaction can be confirmed by general analytical means such as gas chromatography, high performance liquid chromatography, thin-layer chromatography, NMR, and IR.

After the reaction termination, an alcohol compound which is a product is contained in the obtained reaction mixture. An objective alcohol compound can be isolated by performing general isolation operations such as washing, separation, crystallization and condensation on the reaction mixture.

When an insoluble matter such as a ruthenium complex is deposited in the reaction mixture, the above isolation treatment may be performed after removing the insoluble matter by filtration and the like, if needed.

An organic solvent which is not miscible with water may be used for the above separation treatment, if needed. Also, the isolated alcohol compound may be purified by general purifying means such as distillation and column chromatography.

The organic solvent which is not miscible with water herein includes aromatic hydrocarbon solvents such as toluene, xylene and chlorobenzene; aliphatic hydrocarbon solvents such as pentane, hexane and heptane; halogenated hydrocarbon solvents such as dichloromethane, dichloroethane and chloroform; ether solvents such as diethylether and methyl tert-butyl ether; ester solvents such as ethyl acetate; and the like.

An alcohol compound obtained by the above reduction is a compound having -CH₂OH. The -CH₂OH is a group formed by reducing an ester in the carboxylic acid ester compound with hydrogen.

When aliphatic hydrocarbon-containing carboxylic acid esters are used as the carboxylic acid ester compound, the following alcohol compounds are, for example, obtained.

Ethanol, 1-propanol, isopropanol, 1-butanol, isobutanol, tert-butanol, 1-pentanol, cyclopentanol, 1-hexanol, 1-heptanol, 1-octanol, 1-nonanol, 1-decanol, allyl alcohol, ethylene glycol, 1,3-propanediol, 1,4-butanediol, 1,6-hexanediol, 3,3-dimethyl-2-(2-methyl-1-propenyl)cyclopropanemethanol, and 3,3-dimethyl-2-(1-propenyl)cyclopropanemethanol.

When aromatic hydrocarbon-containing carboxylic acid esters are used as the carboxylic acid ester compound, the following alcohol compounds are, for example, obtained.

Benzyl alcohol, 2-fluorobenzyl alcohol, 3-fluorobenzyl alcohol, 4-fluorobenzyl alcohol, 2-chlorobenzyl alcohol, 4-chlorobenzyl alcohol, 4-aminobenzyl alcohol, 4-methoxybenzyl alcohol, 4-methyl-2,3,5,6-tetrafluorobenzyl alcohol, 2,3,5,6-tetrafluorobenzyl alcohol, 2-phenyl-1-ethanol, 4-phenyl-1-butanol, 3-(4-hydroxypnenyl)-1-propanol, 1-naphthylmethanol, 1,2-benzenedimethanol, 1,3-benzenedimethanol, 1,4-benzenedimethanol, 3,4,5,6-tetrafluoro-1,2-benzenedimethanol, and 2,4,5,6-tetrafluoro-1,3-benzenedimethanol.

When cyclic carboxylic acid esters are used as the carboxylic acid ester compound, the following alcohol compounds are, for example, obtained.

1,3-Propanediol, 1,4-butanediol, 1,5-pentanediol, 1,6-hexanediol, 3-methyl-1,6-hexanediol, 4-methyl-1,6-hexanediol, 1,7-heptanediol, 1,8-octanediol, 1,9-nonanediol, and 1,10-decanediol.

When compounds having multiple esters are used as the carboxylic acid ester compound to perform the reaction, alcohol compounds whose one or more esters are reduced with hydrogen are obtained.

When halogen-substituted terephthalic acid diesters are, for example, used as the carboxylic acid ester compound, as obtained alcohol compounds, a compound represented by the formula (7): 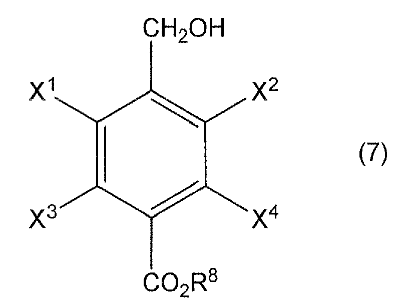 wherein R⁸ represents the same meaning as described above; and X¹, X², X³ and X⁴ each independently represent a hydrogen atom or a halogen atom, with the proviso that at least one of X¹, X², X³ and X⁴ is a halogen atom) (hereinafter may be referred to as Compound (7);
and/or a compound represented by the formula (8) 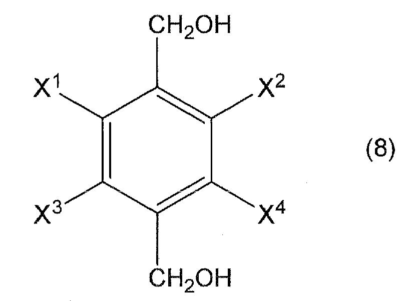 wherein, X², X³ and X⁴ each represent the same meanings as described above (hereinafter may be referred to as Compound (8)) can be obtained.

Examples of Compound (7) include methyl 2-fluoro-(4-hydroxymethyl)benzoate, ethyl 2-chloro-(4-hydroxymethyl)benzoate, methyl 2,5-difluoro-(4-hydroxymethyl)benzoate, propyl 2,6-difluoro-(4-hydroxymethyl)benzoate, methyl 2,3-difluoro-(4-hydroxymethyl)benzoate, methyl 2,5-dichloro(4-hydroxymethyl)benzoate, methyl 2,6-dichloro-(4-hydroxymethyl)benzoate, methyl 2,3-dichloro-(4-hydroxymethyl)benzoate, methyl 2,3,5-trifluoro-(4-hydroxymethyl)benzoate, methyl 2,3,5-trichloro-(4-hydroxymethyl)benzoate, methyl 2,3,5,6-tetrafluoro-(4-hydroxymethyl)benzoate, ethyl 2,3,5,6-tetrafluoro-(4-hydroxymethyl)benzoate, propyl 2,3,5,6-tetrafluoro-(4-hydroxymethyl)benzoate, butyl 2,3,5,6-tetrafluoro-(4-hydroxymethyl)benzoate, methyl 2,3,5,6-tetrachloro-(4-hydroxymethyl)benzoate, and methyl 2,3,5-trifluoro-6-chloro-(4-hydroxymethyl)benzoate.

Examples of Compound (8) include
2-fluoro-1,4-benzenedimethanol,
2-chloro-1,4-benzenedimethanol,
2,5-difluoro-1,4-benzenedimethanol,
2,6-difluoro-1,4-benzenedimethanol,
2,3--difluoro-1,4-benzenedimethanol,
2,5-dichloro-1,4-benzenedimethanol,
2,6-dichloro-1,4-benzenedimethanol,
2,3,-dichloro-1,4-benzenedimethanol,
2,3,5-trifluoro-1,4-benzenedimethanol,
2,3,5-trichloro-1,4-benezenedimethanol,
2,3,5,6-tetrafluorobenenzenedimethanol.
2,3,5,6-tetrachlorobenzenedimethanol, and
2,3,5-trifluoro-6-chlorobenzenedimethanol.

### EXAMPLES

The present invention will be described hereinafter in more detail by way of examples.

The melting point was measured using an automatic melting point measuring apparatus, METTLER FP62, manufactured by Mettler-Toledo International Inc.

NMR was measured using an FT-NMR apparatus, DPX 300, manufactured by Bruker Japan Co., Ltd.

Gas chromatography was measured using GC-17A, manufactured by Shimadzu Corporation.

### Reference Example 1

Into a 100 mL flask equipped with a Dean-Stark reflux condenser, 25 g of 3-(1H-imidazo-1-lyl)-1-propaneamine, 21.2 g of benzaldehyde, 100 mg of p-toluenesulfonic acid and 30 g of toluene were charged, and the resulting mixture was heated, and azeotropic dehydration reaction was performed for 4 hours while refluxing. The resulting reaction mixture was cooled to room temperature (approximately 25°C), washed with 20 g of a 5% by weight solution of potassium carbonate, and then washed with 20 g of water, and the resulting mixture was concentrated to yield 42 g of N-benzylidene-3-(1H-imidazo-1-lyl)-1-propaneamine as a pale yellow oil.

### Reference Example 2

Into a 100 mL flask with a reflux condenser, 5.7 g of N-benzylidene-3-(1H-imidazo-1-lyl)-1-propaneamine obtained from Reference Example 1, 5 g of 1,3,5-trimethyl-2-(chloromethyl)benzene and 20 g of toluene were charged, and the resulting mixture was stirred at 100°C for 5 hours. The obtained reaction mixture was cooled to room temperature, and the obtained oily substance containing 1-[(2,4,6-trimethylphenyl)methyl]-3-(3-aminopropyl)imidazoli um chloride was recovered by decantation. Further, to the oily substance, 20 g of toluene was added and mixed, and an operation in which an oily substance was recovered from the resulting mixture was repeated three times.

Into a flask, 9.7 g of the oily substance obtained from the operation, 20 g of toluene, 20 g of water and 3.3 g of 35% hydrochloric acid were charged, and the obtained mixture was stirred at 80°C for 1 hour. The obtained reaction mixture was cooled to room temperature, and the toluene layer was separated by separation treatment. The resulting aqueous layer was washed twice with 10 g of toluene, and then concentrated to yield 8.8 g of
1-[(2,4,6-trimethylphenyl)methyl]-3-(3-aminopropyl)imidazoli um chloride hydrochloride as white crystals.

Into a 100 mL flask, 8.8 g of the white crystals and 20 g of methanol were charged, and to the mixture, 1.07 g of sodium hydroxide was added, and the obtained mixture was stirred at room temperature for 1 hour. Upon adding 10 g of acetonitrile to the obtained mixture, a salt was deposited. The obtained mixture was filtered to remove the salt, and the obtained filtrate was concentrated to yield 7.8 g of
1-[(2,4,6-trimethylphenyl)methyl]-3-(3-aminopropyl)imidazoli um chloride as a pale yellow oily substance.
1-[(2,4,6-Trimethylphenyl)methyl]-3-(3-aminopropyl)imldazoli um chloride (Pale yellow oily substance)
¹H-NMR (δppm, DMSO-d₆, TMS standard) : 1.90 (m, 2H), 2.23 (s, 3H), 2.25 (s, 6H), 2.52 (m, 2H), 4.29 (m, 2H), 5.48 (s, 2H), 6.99 (s, 2H), 7.63 (s, 1H), 7.94 (s, 1H), 9.23 (s, 1H)

### Reference Example 3

Into a 100 mL flask equipped with a reflux condenser, 12.6 g of N-benzylidene-3-(1H-imidazo-1-lyl)-1-propaneamine obtained from Reference Example 1, 12 g of benzylbromide and 50 g of toluene were charged, and the obtained mixture was stirred at 100°C for 5 hours. The obtained reaction mixture was cooled to room temperature, and the obtained oily substance containing 1-benzyl-3-(3-aminopropyl)imidazolium bromide was recovered by decantation. Further, to the oily substance, 20 g of toluene was added and mixed, and an operation in which an oily substance was recovered from the resulting mixture was repeated three times.

The oily substance obtained from the operation (22.7 g), 50 g of toluene, 50 g of water and 7.5 g of 35% hydrochloric acid were charged, and the obtained mixture was stirred at 80°C for 1 hour. The obtained reaction mixture was cooled to room temperature, and the toluene layer was separated by separation treatment. The obtained aqueous layer was washed twice with 10 g of toluene, and then concentrated to yield 19.9 g of 1-benzyl-3-(3-aminopropyl)imidazolium bromide hydrochloride as white crystals.

Into a 100 mL flask, 19.4 g of the white crystals obtained above and 50 g of methanol were charged, and 2.33 g of sodium hydroxide was added to the mixture, and the obtained mixture was stirred at room temperature for 1 hour. Upon adding 30 g of acetonitrile to the obtained mixture, a salt was deposited. The obtained mixture was filtered to remove the salt and the obtained filtrate was concentrated to yield 17.3 g of
1-benzyl-3-(3-aminopropyl)imidazolium bromide as a pale yellow oily substance.
1-Benzyl-3-(3-aminopropyl)imidazolium bromide (Pale yellow oily substance)
¹H-NMR (δppm, DMSO-d₆, TMS standard) : 1.86 (m, 2H), 2.48 (m, 2H), 4.29(s, 3H), 2.25 (s, 6H), 3.56 (m, 2H), 4.29 (m, 2H), 5.50 (s, 2H), 7.2-7.5 (m, 5H), 7.89 (m, 2H), 9.54 (s, 1H)

### Reference Example 4

Into a 100 mL flask equipped with a reflux condenser, 5 g of 1-methylimidazole, 9.6 g of 3-dimethylamino-1-chloropropane hydrochloride and 50 g of acetonitrile were charged, and the obtained mixture was stirred at 80°C for 16 hours. The obtained reaction mixture was cooled to room temperature and then filtered to remove crystals, and the crystals were dried to yield 6.5 g of 3-[(3-dimethylamino)propyl]-1-methyl-1H-imidazolium chloride hydrochloride.

### Example 1

Into a 50 mL flask equipped with a refiux condenser, 300 mg of 1-[(2,4,6-trimethylphenyl)methyl]-3-(3-aminopropyl)imidazoli um chloride obtained from Reference Example 2 and 10 g of tetrahydrofuran were charged, and the inside of the flask was replaced with nitrogen. Thereto, 45 mg of sodium hydride-mineral oil dispersion (content 60%) was added, and this was stirred at 25°C for 30 minutes. To the mixture, 150 mg of a (p-cymene) ruthenium chloride dimer was added, and the obtained mixture was stirred at 40°C for 8 hours, and then the obtained reaction mixture was cooled to room temperature. The reaction mixture was filtered to remove a salt, and the obtained filtrate was concentrated to yield 290 g of a black green powder containing a ruthenium complex. The melting point of the powder was 245° to 250°C (decomposition).

### Example 2

Into a 100 mL autoclave equipped with a glass cylinder, 50 mg of the black green powder obtained from Example 1, 20 mg of potassium hydroxide, 200 mg of methyl benzoate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 100°C, the inner pressure became 1.3 MPa. Upon stirring the contents of the autoclave at 100°C for 16 hours, the inner pressure became 1.2 MPa. When the contents of the autoclave were cooled to room temperature (approximately 25°C) and analyzed by gas chromatography (internal standard method), the yield of benzyl alcohol was 10%. Also, methyl benzoate recovery was 85%.

### Example 3

Into a 50mL flask equipped with a reflux condenser, 240 mg of 3-[(3-dimethylamino)propyl]-1-methyl-1H-imidazolium chloride hydrochloride synthesized in Reference Example 4 and 10 g of tetrahydrofuran were charged, and the inside of the flask was replaced with nitrogen. Thereto, 80 mg of sodium hydride-mineral oil dispersion (content 60%) was added, and this was stirred at 25°C for 30 minutes. To the mixture, 470 mg of tris(triphenylphosphine) ruthenium dichloride was added, and the obtained mixture was stirred at 40°C for 8 hours, and then the resulting reaction mixture was cooled to room temperature (25°C). The reaction mixture was filtered to remove a salt, and the obtained filtrate was concentrated to yield 590 mg of a black green powder containing a ruthenium complex. The melting point of the powder was 220° to 225°C (decomposition).

### Example 4

Into a 100 mL autoclave equipped with a glass cylinder, 50 mg of the black green powder obtained from Example 3, 10 mg of potassium hydroxide, 200 mg of dimethyl 2,3,5,6-tetrafluoroterephthalate and 10 g of toluene were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1.5 MPa. When the contents of the autoclave were cooled to room temperature and analyzed by gas chromatography (internal standard method), the yield of 2,3,5,6-tetrafluorobenzenedimethanol was 5% and the yield of methyl 2,3,5,6-tetrafluoro-4-hydroxymethyl benzoate was 42%. Also, dimethyl 2,3,5,6-tetrafluoroterephthalate recovery was 22%.

### Example 5

Into a 50mL flask equipped with a reflux condenser, 720 mg of 3-[(3-dimethylamino)propyl]-1-methyl-1H-imidazolium chloride hydrochloride synthesized in Reference Example 4 and 20 g of tetrahydrofuran were charged, and the inside of the flask was replaced with nitrogen. Thereto, 250 mg of sodium hydride-mineral oil dispersion (content 60%) was added, and this was stirred at 25°C for 30 minutes. To the mixture, 1410 mg of tris(triphenylphosphine) ruthenium dichloride was added, and the obtained mixture was stirred at 40°C for 2 hours, and then the resulting reaction mixture was filtered to remove a salt and cooled to 0°C, and the mixture was left to stand at 0°C for 10 hours. After cooling, deposited crystals were filtered, and dried to yield 590 mg of a black green powder containing a ruthenium complex.
Ruthenium complex (containing black green powder)
¹H-NMR (δppm, DMSO-d₆, TMS standard) : 2.35 (m, 2H), 2.55(S, 6H), 3.1-3.5 (m, 4H), 3.65(s, 3H), 7.70 (s, 1H), 7.80 (s, 2H) (a peak identified as triphenylphosphine (around 6.95) was not found.)

### Example 6

Into a 100 mL autoclave equipped with a glass cylinder, 40 mg of the black green powder obtained from Example 5, 10 mg of sodium hydride, 200 mg of y-butyrolactone and 4 g of tetrahydrofuran were charged. The inside of the autoclave was replaced with nitrogen and then replaced with hydrogen, followed by increasing the hydrogen pressure to 1.0 MPa. Then, upon increasing the temperature to 120°C, the inner pressure became 1.4 MPa. Upon stirring the contents of the autoclave at 120°C for 6 hours, the inner pressure became 1.2 MPa. When the contents of the autoclave were cooled to approximately 25°C and analyzed by gas chromatography (internal standard method), the yield of 1, 4-butanediol was 14%. Also, γ-butyrolactone recovery was 85%.

### Example 7

Into a 50 mL flask equipped with a reflux condenser, 510 mg of 1-[(2,4,6-trimethylphenyl)methyl]-3-(3-aminopropyl)imidazoli um chloride obtained from Reference Example 2 and 10 g of dimethylacetoamide were charged, and to the mixture, 243 mg of dichloro(1,5-cyclooctadiene)ruthenium was added, and the inside of the flask was replaced with nitrogen. Thereto, 70 mg of sodium hydride-mineral oil dispersion (content 60%) was added, and the resulting mixture was stirred at 25°C for 1 hour. The obtained mixture was filtered to remove a salt, and the obtained filtrate was concentrated and dried to yield 510 mg of black green crystals containing a ruthenium complex.

### Example 8

Into a 100 mL autoclave equipped with a glass cylinder, 50 mg of the black green crystals obtained from Example 7, 5 mg of sodium hydride, 200 mg of γ-butyrolactone and 4 g of tetrahydrofuran were charged. The inside of the autoclave was replaced with nitrogen and then replaced with hydrogen, followed by increasing the hydrogen pressure to 1.0 MPa. Then, upon increasing the temperature to 120°C, the inner pressure became 1.4 MPa. Upon stirring the contents of the autoclave at 120°C for 6 hours, the inner pressure became 1.3 MPa. When the contents of the autoclave were cooled to room temperature and analyzed by gas chromatography (internal standard method), the yield of 1, 4-butanediol was 15%. Also, γ-butyrolactone recovery was 84%.

### Example 9

Into a 100 mL autoclave equipped with a glass cylinder, 50 mag of the black green crystals obtained from Example 7, 5 mg of sodium hydride, 200 mg of methyl 3-phenylpropionate and 4 g of toluene were charged. The inside of the autoclave was replaced with nitrogen and then replaced with hydrogen, followed by increasing the hydrogen pressure to 1.0 MPa. Then, upon increasing the temperature to 120°C, the inner pressure became 1.2 MPa. Upon stirring the contents of the autoclave at 120°C for 6 hours, the inner pressure became 1.16 MPa. When the contents of the autoclave were cooled to room temperature and analyzed by gas chromatography (internal standard method), the yield of 3-phenylpropanol was 5%. Also, methyl 3-phenylpropionate recovery was 90%.

### Example 10

Into a 50 mL flask equipped with a reflux condenser, 260 mg of 1-[(2,4,6-trimethylphenyl)methyl]-3-(3-aminopropyl)imidazoli um chloride obtained from Reference Example 2 and 10 g of dimethylacetoamide were charged, and to the mixture, 92 mg of ruthenium trichloride was added, and the inside of the flask was replaced with nitrogen. Thereto, 40 mg of sodium hydride-mineral oil dispersion (content 60%) was added, and the resulting mixture was stirred at 25°C for 1 hour. The obtained mixture was filtered to remove a salt, and upon adding dropwise the obtained filtrate to 100 g of nitrogen-substituted toluene, black green crystals were deposited. The crystals were filtered and dried to yield 220 g of a black green powder containing a ruthenium complex.
Ruthenium complex (Black green powder)
¹H-NMR (δppm, DMSO-d₆, TMS standard): 1.95 (m, 2H), 2.30 (s, 3H), 2.59 (s, 6H), 4.1-4.5 (m, 2H), 5.45 (s, 2H), 6.85 (bs, 2H), 7.1-7.3 (m, 2H), 7.65(m, 2H)

### Example 11

Into a 100 mL autoclave equipped with a glass cylinder, 11 mg of the black green powder obtained from Example 10, 5 mg of sodium hydride, 200 mg of γ-butyrolactone and 4 g of tetrahydrofuran were charged. The inside of the autoclave was replaced with nitrogen and then replaced with hydrogen, followed by increasing the hydrogen pressure to 1.0 MPa. Then, upon increasing the temperature to 120°C, the inner pressure became 1.4 MPa. Upon stirring the contents of the autoclave at 120°C for 6 hours, the inner pressure became 1.3 MPa. When the contents of the autoclave were cooled to room temperature and analyzed by gas chromatography (internal standard method), the yield of 1,4-butanediol was 14%. Also, γ-butyrolactone recovery was 85%.

### Example 12

Into a 50 mL flask equipped with a reflux condenser, 390 mg of 1-benzyl-3-(3-aminopropyl)imidazolium bromide obtained from Reference Example 3 and 10 g of dimethylacetoamide were charged, and to the mixture, 138 mg of ruthenium trichloride was added, and the inside of the flask was replaced with nitrogen. Thereto, 60 mg of sodium hydride-mineral oil dispersion (content 60%) was added, and the obtained mixture was stirred at 25°C for 2 hours. The obtained mixture was filtered to remove a salt, and upon adding dropwise the obtained filtrate to 100 g of nitrogen-substituted toluene, black green crystals were deposited. The crystals were filtered and dried to yield 390 mg of a black green powder containing a ruthenium complex.
¹H-NMR (δppm, DMSO-d₆, TMS standard) : 1.93 (m, 2H), 2.48 (s, 3H), 4.30(m, 2H), 5.50(m, 2H), 7.1-7.5(m, 5H), 7.99(m, 2H)

### Example 13

Into a 100 mL autoclave equipped with a glass cylinder, 20 mg of the black green powder obtained from Example 12, 5 mg of sodium hydride-mineral oil dispersion (content 60%), 200 mg of γ-butyrolactone and 4 g of tetrahydrofuran were charged. The inside of the autoclave was replaced with nitrogen and then replaced with hydrogen, followed by increasing the hydrogen pressure to 1.0 MPa. Then, upon increasing the temperature to 120°C, the inner pressure became 1.4 MPa. Upon stirring the contents of the autoclave at 120°C for 6 hours, the inner pressure became 1.3 MPa. When the contents of the autoclave were cooled to room temperature and analyzed by gas chromatography (internal standard method), the yield of 1,4-butanediol was 22%. Also, γ-butyrolactone recovery was 77%.

### (Comparative Example 1)

In Example 4, the same reactions as in Example 2 were repeated except that 50 mg of tris (triphenylphosphine) ruthenium dichloride was used in place of 50 mg of the black green powder obtained from Example 3. When the contents of the autoclave were analyzed by gas chromatography (internal standard method), the generation of 2,3,5,6-tetrafluorobenzenedimethanol and the generation of methyl 2,3,5,6-tetrafluoro-4-hydroxymethyl benzoate were not found, and 2,3,5,6-tetrafluoroterephthalic acid dimethyl ester recovery was 90%.

### (Comparative Example 2)

Into a 100 mL autoclave equipped with a glass cylinder, 20 mg of bis{2-[bis(1,1-dimethylethyl)phosphino-κP]ethaneamine-κN}dic hlororuthenium (obtained from Aldrich), 20 mg of potassium hydroxide, 400 mg of dimethyl 2,3,5,6-tetrafluoroterephthalate and 20 g of toluene were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1.5 MPa. When the contents of the autoclave were cooled to room temperature and analyzed by gas chromatography (internal standard method), the yield of 2,3,5,6-tetrafluorobenzenedimethanol was 8% and the yield of methyl 2,3,5,6-tetrafluoro-4-hydroxymethyl benzoate was 51%. Also, dimethyl 2,3,5,6-tetrafluoroterephthalate recovery was 22%.

### (Comparative Example 3)

Into a 100 mL autoclave equipped with a glass cylinder, 20 mg of a (p-cymene) ruthenium chloride dimer, 10 mg of sodium methoxide, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1.6 MPa. When the contents of the autoclave were cooled to room temperature and analyzed by gas chromatography (internal standard method), the generation of 2,3,5,6-tetrafluorobenzenedimethanol and the generation of methyl 2,3,5,6-tetrafluoro-4-hydroxymethyl benzoate were not found, and dimethyl 2, 3, 5, 6-tetrafluoroterephthalate was mainly recovered. Besides, dimethyl 2,3,5-trifluoroterephthalate, methyl 2,3,5,6-tetrafluorobenzoate and the like were found to be produced as by-products.

### (Comparative Example 4)

Into a 100 mL autoclave equipped with a glass cylinder, 100 mg of 5% palladium/carbon, 200 mg of dimethyl 2,3,5,6-tetrafluoroterephthalate and 10 g of tetrahydrofuran were charged. The inside of the autoclave was replaced with nitrogen and then replaced with hydrogen, followed by increasing the hydrogen pressure to 1.0 MPa. Then, upon increasing the temperature to 170°C, the inner pressure became 1.6 MPa. Upon stirring the contents of the autoclave at 170°C for 4 hours, the inner pressure became 1. 6 MPa. When the contents of the autoclave were cooled to room temperature and analyzed by gas chromatography (internal standard method), the generation of 2,3,5,6-tetrafluorobenzenedimethanol and the generation of methyl 2,3,5,6-tetrafluoro-4-hydroxymethyl benzoate were not found, and dimethyl 2,3,5,6-tetrafluoroterephthalate was mainly recovered. Besides, dimethyl 2,3,5-trifluoroterephthalate, methyl 2,3,5,6-tetrafluorobenzoate and the like were found to be produced as by-products.

### INDUSTRIAL APPLICABILITY

According to the present invention, it is possible to provide a production method, wherein a carboxylic acid ester compound can be reduced to an alcohol compound, and a suitable catalyst for the production method.

## Claims

1. A method for producing an alcohol compound, wherein a carboxylic acid ester compound is reduced with hydrogen in the presence of a ruthenium complex which is obtained by reacting an imidazolium salt (A) having at least one optionally substituted amino group with a ruthenium compound (B) in the presence of a base (C).

2. The production method according to claim 1, wherein in the imidazolium salt (A), the optionally substituted amino group is bound to a nitrogen atom in an imidazolium ring through a linking group.

3. The production method according to claim 2, wherein the linking group is an optionally substituted alkylene group.

4. The production method according to claim 1, wherein the imidazolium salt (A) has a group represented by the formula (1) : 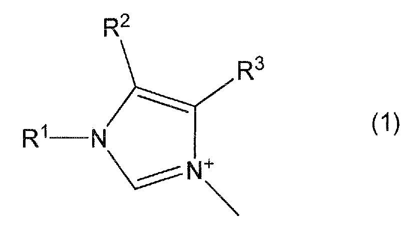 wherein R¹ represents an optionally substituted alkyl group or an optionally substituted aryl group; R² and R³ each independently represent a hydrogen atom, an optionally substituted alkyl group or an optionally substituted aryl group, or R² and R³ are together with the carbon atoms to which R² and R³ each are bound to represent a ring.

5. The production method according to claim 1, wherein the imidazolium salt (A) has a group represented by the formula (2), as an optionally substituted amino group, 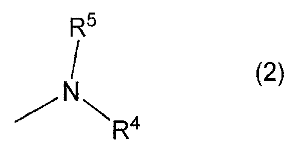 wherein R⁴ and R⁵ each independently represent a hydrogen atom or an alkyl group having 1 to 4 carbon atoms, or R⁴ and R⁵ are taken together to represent an alkylene group having 2 to 8 carbon atoms.

6. The production method according to claim 1, wherein the imidazolium salt (A) is represented by the formula (3): 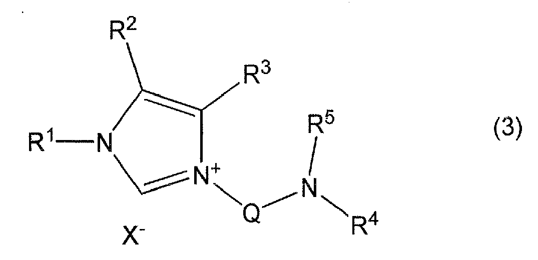 wherein R¹ represents an optionally substituted alkyl group or an optionally substituted aryl group; R² and R³ each independently represent a hydrogen atom, an optionally substituted alkyl group or an optionally substituted aryl group, or R² and R³ are taken together to represent a ring; R⁴ and R⁵ each independently represent a hydrogen atom or an alkyl group having 1 to 4 carbon atoms, or R⁴ and R⁵ are taken together to represent an alkylene group having 2 to 8 carbon atoms. Q represents an optionally substituted alkylene group, and X⁻ represents a monovalent anion).

7. The production method according to claim 1, wherein the ruthenium compound (B) contains halogens.

8. The production method according to claim 1, wherein the ruthenium compound (B) is at least one member selected from the group consisting of a compound comprised of halogens and ruthenium, an aromatic compound-coordinated ruthenium dihalide dimer, a diene-coordinated ruthenium dihalide polymer, and a tris(triphenylphosphine)ruthenium compound.

9. The production method according to claim 1, wherein the base (C) is at least one member selected from the group consisting of an alkali metal alkoxide, an alkali metal hydride and an alkali metal bis(trialkylsilyl)amide.

10. The production method according to claim 1, wherein the carboxylic acid ester compound is a carboxylic acid ester compound having an aliphatic hydrocarbon group, a carboxylic acid ester compound having an aromatic hydrocarbon group, or a cyclic carboxylic acid ester compound.

11. The production method according to claim 1, wherein the carboxylic acid ester compound is a compound represented by the formula (6): 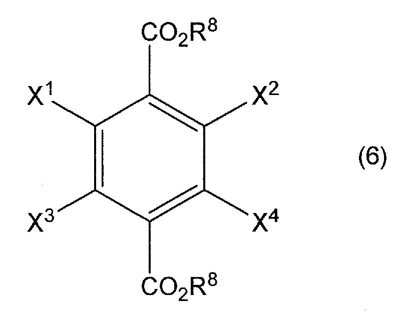 wherein R⁸ represents an alkyl group having 1 to 6 carbon atoms; and X¹, X², X³ and X⁴ each independently represent a hydrogen atom or a halogen atom, with the proviso that at least one of X¹, X², X³ and X⁴ is a halogen atom.

12. The production method according to claim 1, wherein the amount to be used of the ruthenium complex, which is converted into the amount of ruthenium atoms, is in the range of 0.001 to 0.2 mol per mol of ester of the carboxylic acid ester compound.

13. The production method according to claim 1, wherein the carboxylic acid ester compound is reduced with hydrogen in the presence of a base.

14. A ruthenium complex which is obtained by reacting an imidazolium salt (A) having at least one optionally substituted amino group with a ruthenium compound (B) in the presence of a base (C).

15. A composition containing a ruthenium complex which is obtained by reacting an imidazolium salt (A) having at least one optionally substituted amino group with a ruthenium compound (B) in the presence of a base (C).
